# EUROPEAN PATENT APPLICATION

(11) **EP 4 342 467 A1**
(43) Date of publication of application: **27.03.2024**
(21) Application number: 22196503.1
(22) Date of filing: 20.09.2022
(51) Int. Cl.: A61K 31/426, A61K 31/427, A61K 31/4439, A61K 31/444, A61K 45/06, A61K 31/196, A61K 33/243, A61K 38/14, A61P 35/00

(54) **KU INHIBITORS FOR USE IN TUMOR THERAPY**

(71) Applicant: Masarykova Univerzita, 60177 Brno (CZ)
(72) Inventor: Fulnecek, Jaroslav, Brno (CZ); Riha, Karel, Ivan (CZ)
(74) Representative: Harber IP s.r.o.

(57) **Abstract**

The invention provides compounds of general formula I 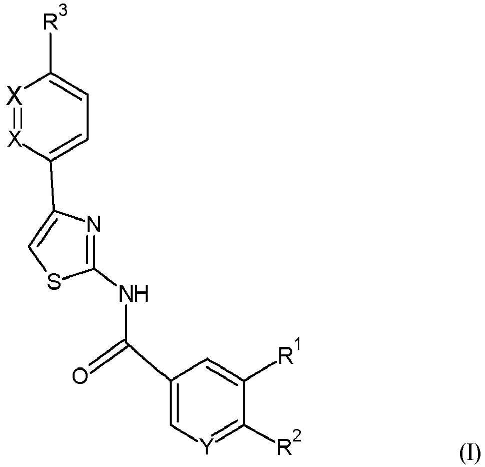 wherein
each of X is CH or N, wherein at most one X is N;
Y is CH or N;
R¹ is selected from H, halogen;
R² is selected from H, C1-C4 alkyl, C1-C4 alkylamino, C3-C6 cykloalkylamino;
R³ is selected from H, C1-C4 alkyl, -(CH₂)ₙ-NH-C(O)-(C1-C4 alkyl) wherein n=0 to 4;,
or pharmaceutically acceptable salts or solvates thereof,
for use in the treatment of tumor diseases.

## Description

### Field of Art

The present invention relates to heterocyclic compounds useful as inhibitors of binding of Ku to DNA.

### Background Art

Genome instability is one of the most pervasive characteristics of tumor cells. Genome instabilities drive tumor evolution and mutations affecting DNA damage response and repair (DDR) can be hitchhiked with mutations that were selected for tumor growth and propagation (Lord, C.J., and Ashworth, A. (2012). The DNA damage response and cancer therapy. Nature 481, 287-294). Therefore, DDR is often altered in tumors. This has two major implications for cancer therapy. First, conventional cancer treatments such as chemotherapy or radiotherapy usually implement their effect by damaging the DNA. Upregulation of DDR pathways enhances the ability of tumor cells to circumvent catastrophic DNA damage and contributes to chemo- and radiotherapy resistance. Therefore, drugs targeting DDR are expected to block this resistance and sensitize tumor cells to chemo- and radiotherapy. Second, many DDR mechanisms act redundantly and some DNA lesions can be repaired by multiple pathways, meaning that deficiency in one pathway does not necessarily impair cell growth. This feature can be exploited to selectively target tumor cells with altered DDR through synthetic lethality. The most successful application of synthetic lethality in cancer therapy takes advantage of such synergism between DNA repair pathways for treatment of BRCA1/2-mutated tumor cells using PARP inhibitors (Kim, G. et al. (2015). FDA Approval Summary: Olaparib Monotherapy in Patients with Deleterious Germline BRCA-Mutated Advanced Ovarian Cancer Treated with Three or More Lines of Chemotherapy. Clin Cancer Res 21, 4257-4261). Thus, targeting DDR can lead to selective chemotherapeutic drugs that specifically suppress tumor cells lowering adverse side effects associated with treatments.

Ku is a DNA-end binding complex primarily known for its function in DNA repair pathway called non-homologous end joining (NHEJ) (Zhao, B., Rothenberg, E., Ramsden, D.A., and Lieber, M.R. (2020). The molecular basis and disease relevance of non-homologous DNA end joining. Nat Rev Mol Cell Biol 21, 765-781). It is an abundant nuclear complex that binds free DNA ends in sequence unspecific manner, and acts as a sensor for DNA double strand breaks. Upon DNA binding, Ku protects broken DNA ends from degradation and recruits and activates DNA-dependent protein kinase, catalytic subunit (DNA-PKcs), a kinase that phosphorylates downstream factors of NHEJ and the DDR cascade. Ku also forms a synaptic complex with XRCC4 and ligase IV that bridges DSB aligning the two broken DNA termini. In next step, Ku translocates away from DNA termini freeing the ends for processing and religation. NHEJ is the major DNA repair pathway that repairs majority of DSBs throughout the cell cycle and cellular deficiency in NHEJ factors leads to increased sensitivity to genotoxic agents. Nevertheless, Ku function is not limited to NHEJ. It is also implicated in controlling resection of single-ended DSBs arising from arrested replication forks during S-phase (Balestrini, A., Ristic, D., Dionne, I., Liu, X.Z., Wyman, C., Wellinger, R.J., and Petrini, J.H. (2013). The Ku heterodimer and the metabolism of single-ended DNA double-strand breaks. Cell Rep 3, 2033-2045). Furthermore, Ku protects telomeres from unwanted recombination. Ku-deficiency causes excision of telomeric circular molecules (t-circles) via intrachromatid recombination resulting in deletion of large parts of telomeric DNA (Zellinger, B., Akimcheva, S., Puizina, J., Schirato, M., and Riha, K. (2007). Ku suppresses formation of telomeric circles and alternative telomere lengthening in Arabidopsis. Mol Cell 27, 163-169). Knock-out of Ku in human cell lines is lethal, which is attributed to the rapid deletion of telomeres via t-circle excision (Wang, Y., Ghosh, G., and Hendrickson, E.A. (2009). Ku86 represses lethal telomere deletion events in human somatic cells. Proc Natl Acad Sci U S A 106, 12430-12435).

Considering its central role in DNA repair, NHEJ is an interesting target in tumor therapy. Inhibition of NHEJ is expected to synergize effect of DNA damaging drugs and radiotherapy, and induce synthetic lethality in homologous recombination-deficient cancers. So far, most effort was focused on DNA-PKcs. Several potent inhibitors of DNA-PKcs are currently tested in clinical trials, mostly in combination with other chemotherapeutics or with radiotherapy (Damia, G. (2020). Targeting DNA-PK in cancer. Mutat Res 821, 111692; Medova, M., Medo, M., Hovhannisyan, L., Munoz-Maldonado, C., Aebersold, D.M., and Zimmer, Y. (2020). DNA-PK in human malignant disorders: Mechanisms and implications for pharmacological interventions. Pharmacol Ther 215, 107617). Inhibitors targeting other NHEJ factors such as ligase IV, XRCC4 and Artemis have been reported, but they are still in early stages of development (Kelm et al. (2022). Recent Advances in the Development of Non-PIKKs Targeting Small Molecule Inhibitors of DNA Double-Strand Break Repair. Front Oncol 12, 850883).

In contrast to DNA-PKcs, relatively little attention has been paid to pharmacological targeting of Ku. Up to date, two types of Ku inhibitors were reported. Computational screening of chemical libraries combined with in silico docking identified STL127705 as inhibitor of Ku-DNA binding (Weterings, E., Gallegos, A.C., Dominick, L.N., Cooke, L.S., Bartels, T.N., Vagner, J., Matsunaga, T.O., and Mahadevan, D. (2016). A novel small molecule inhibitor of the DNA repair protein Ku70/80. DNA Repair (Amst) 43, 98-106). However, this compound confers enhanced sensitivity to irradiation at relatively high concentrations (20-30 µM). The second class of inhibitors was identified by highthroughput screening of chemical libraries for Ku-DNA binding. The identified hit and several of its derivatives were reported to sensitize cells to genotoxic drugs and decreases NHEJ in vivo, but they are biologically active from ∼20 µM concentrations (Gavande et al. (2020). Discovery and development of novel DNA-PK inhibitors by targeting the unique Ku-DNA interaction. Nucleic Acids Res 48, 11536-11550). Two further groups of Ku inhibitors were recently reported in WO 2021/011778 and US 2022/0089577, with Ku inhibitory activities expressed as IC50 ranging in tens to hundreds µM, only a few of them reaching about 5 µM.

### Disclosure of the Invention

The present invention provides Ku inhibitors of general formula I 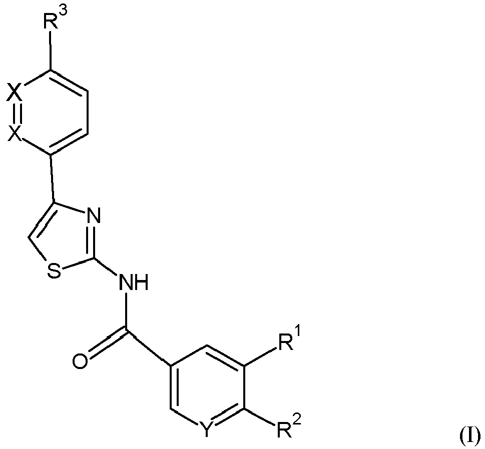 wherein
each of X is CH or N, wherein at most one X is N;
Y is CH or N;
R¹ is selected from H, halogen;
R² is selected from H, C1-C4 alkyl, C1-C4 alkylamino, C3-C6 cykloalkylamino;
R³ is selected from H, C1-C4 alkyl, -(CH₂)ₙ-NH-C(O)-(C1-C4 alkyl) wherein n=0 to 4;
or pharmaceutically acceptable salts or solvates thereof,
for use in the treatment of tumor diseases.

Preferably, at least one of X and Y is N. More preferably, one X is N and Y is N.

Preferably, R¹ is selected from H, Br.

Particularly preferably, R¹ is Br and Y is N.

Preferably, R² is selected from H, methyl, C1-C3 alkylamino, C3 cycloalkylamino.

Preferably, R³ is selected from H, -NH-C(O)-CH₃, -(CH₂)₂-NH-C(O)-CH₃.

In a preferred embodiment, the compound of formula I is selected from 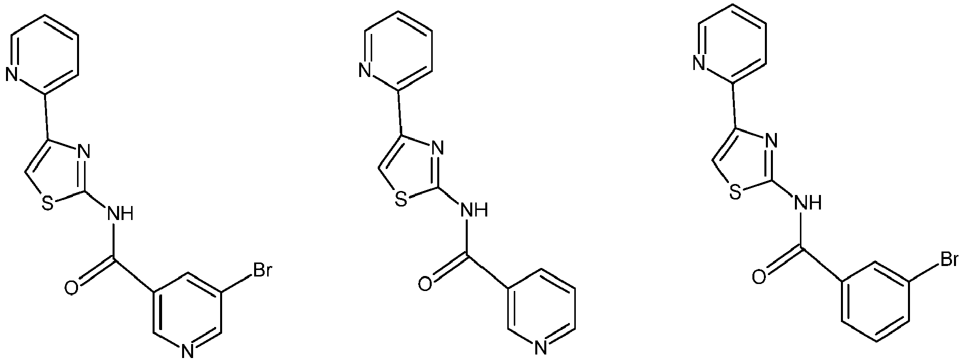 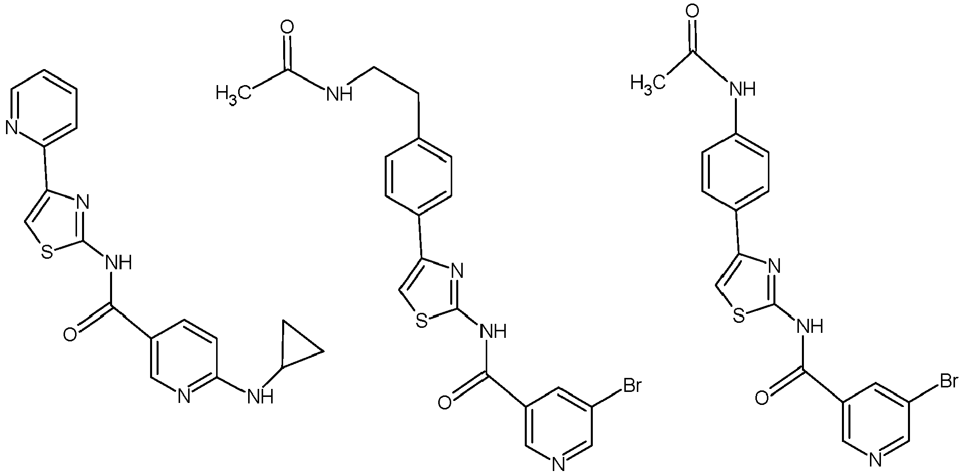 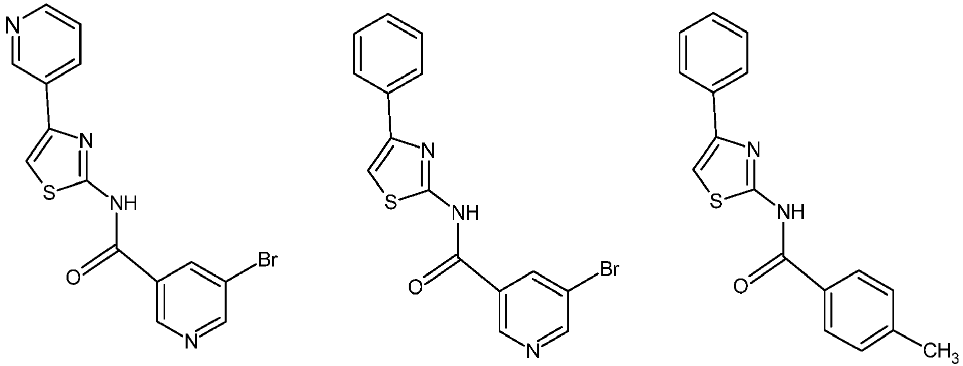 or pharmaceutically acceptable salts or solvates thereof.

Particularly preferably, the compound of formula I is 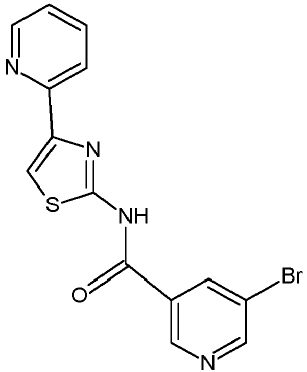 or pharmaceutically acceptable salts or solvates thereof.

Within the framework of the invention, it was found that the compounds of formula I are potent inhibitors of Ku and specifically inhibits association of Ku with DNA. They are capable of inducing rapid telomere shortening through excision of extrachlomosomal telomeric circles, as well as suppressing growth of tumor cells. They are also capable of selective suppression of tumor cells with susceptible genetic background, such as cis-platin resistant SKOV3 cells, or cells deficion in aspects of DNA damage repair and response.

The term "tumor disease" refers to cancers, preferably solid tumors. Solid tumors include, for example, colon cancer, cervical cancer, osteosarcoma, ovarian cancer, prostate cancer, liver cancer, kidney cancer, GIT cancers, squamous cell carcinoma, adenocarcinoma, breast cancer and others.

In some embodiments, the compounds of formula I may be used for treatment of solid tumors wherein the tumor cells have susceptible genetic background. This includes tumors with altered DNA damage response, which are typically tumors resistant to treatments with DNA damaging agents (bleomycin, cisplatin, topoisomerase II inhibitors) or to radiotherapy, or tumors with mutations in genes of DNA damage response or repair (e.g. BRCA1/2, ATM, TP53).

In some embodiments, the present invention provides a compound of formula I in combination with radiotherapy or chemotherapy for use in the treatment of tumor diseases.

Radiotherapy is preferably applied sequentially to the administration of the compound of formula I. More precisely, radiotherapy is preferably applied after the administration of the compound of formula I.

Chemotherapy and the compound of formula I can be applied simultaneously or sequentially. In some embodiments, the chemotherapeutic and the compound of formula I may be contained in one pharmaceutical formulation. In other embodiments, the chemotherapeutic and the compound of formula I may be contained in separate pharmaceutical formulations.

Particularly suitable chemotherapeutics for such combination treatment include DNA damage inducers, telomerase inhibitors, drugs inducing replication stress, inhibitors of other proteins involved in DNA repair and DNA damage response. More specifically, such chemotherapeutics include BIBR1532, bleomycin, cis-platin, topoisomerase II inhibitors.

Compounds of formula I may be formulated in pharmaceutical compositions containing at least one compound of formula I and at least one pharmaceutically acceptable excipient. The excipients typically include binders, fillers, solvents, preservatives, disintegrants, glidants. In some embodiments, the pharmaceutical composition may be a liquid pharmaceutical composition, such as solution for injections or infusion solution. In some embodiments, the pharmaceutical composition may be solid, such as a tablet, a coated tablet or a capsule.

### Brief Description of Drawings

Figure 1: Comparison of the effect of iKU-1 and STL1127705 on Ku-DNA binding by electrophoretic mobility shift assay (Example 1).
Figure 2: Inhibition of DNA-PKcs by iKU-1 *in vivo* (Example 2).
Figure 3: Induction of DNA damage in HTC116 cells by iKU-1 (Example 3).
Figure 4: Induction of extrachromosomal circles by iKU-1 (Example 4).
Figure 5: iKU-1 induces loss of telomeres at subset of chromosome ends (Example 5).
Figure 6: iKU-1 induces rapid shortening of telomeres (Example 5).
Figure 7: Effect of iKU-1 on growth of tumor cell lines (Example 6)
Figure 8: iKU-1 causes cell cycle arrest in S-phase (Example 7).
Figure 9: iKU-1 exhibit increased toxicity in SKOV-3 ovarian cancer line (Example 10).

### Examples

The compounds were obtained from commercial sources (companies Enamine, Chembridge). The formulas and designations of the compounds tested in the examples are as follows:

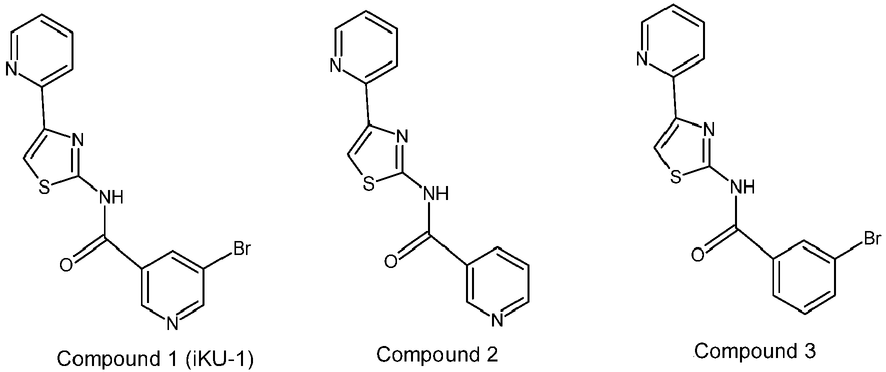

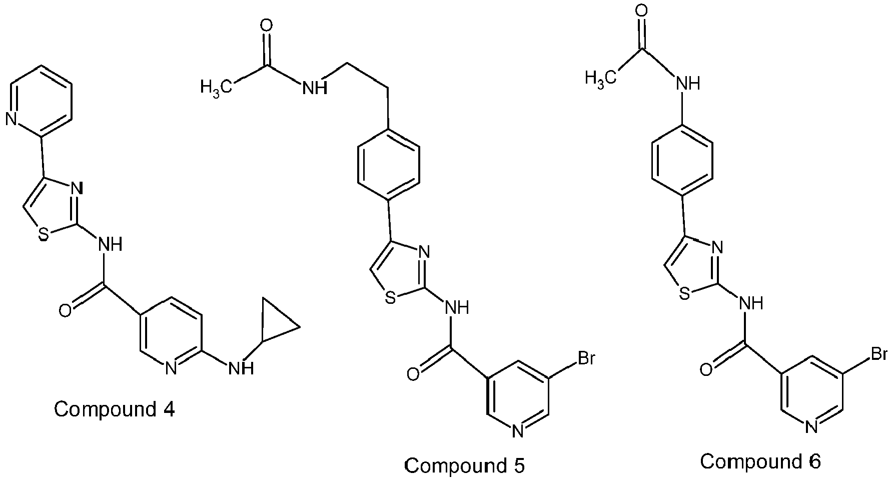

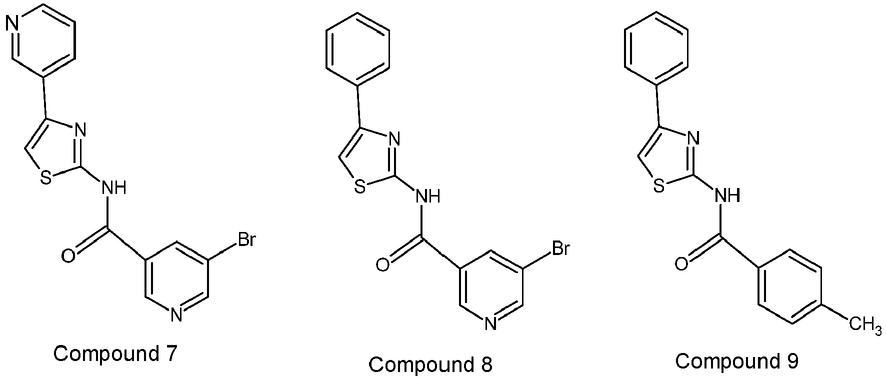

### Example 1

Effect of Compounds 1-9 on Ku-DNA binding was measured by microwell protein induced fluorescent enhancement assay (mwPIFE) as described in Valuchova, S., Fulnecek, J., Petrov, A.P., Tripsianes, K., and Riha, K. (2016). A rapid method for detecting protein-nucleic acid interactions by protein induced fluorescence enhancement. Sci Rep 6, 39653. mwPIFE is based on enhanced fluorescence of cyanine fluorophores attached to nucleic acid probes upon protein binding. We used as a probe 25 bp DNA fragment with Cyanine-3 fluorophore attached to 5' end generated by annealing two oligonucleotides (5'-[Btn]CACAACGACCACCTAGATGGATCCA-3' (SEQ ID NO: 1) and 5'-[Cy3]TGGATCCATCTAGGTGGTCGTTGTG-3' (SEQ ID NO: 2)). The probe was immobilized in 96 well plates through biotin (Btn) attached to one end of DNA and incubated with purified Ku complex (0.5 pmol) in 50 µl of binding buffer (10 mM calcium acetate, 60 mM potassium acetate, 20 mM TRISacetate pH 7.9, 100 mg BSA/1). Ku-DNA binding was calculated from altered fluorescence measured by a fluorescence plate reader (Valuchova et al., 2016). Effect of inhibitors on Ku-DNA binding are presented in Table 1 as IC₅₀ values estimated from the 0.5 to 8 µM concentration range, and percentage of Ku-DNA binding in the presence of 8 µM inhibitor compared to 0.5% DMSO.

**Table 1**

| Compound No. | Inhibition of Ku-DNA binding IC₅₀ [µM]^{∗} | % binding at 8 µM (100% = 0.5% DMSO)^{∗} | Inhibition of cell proliferation IC₅₀ [µM]^{∗∗} | Concentration inducing c-circle formation [µM]^{∗∗∗} |
|---|---|---|---|---|
| 1 (iKu-1) | 3 | 5 | 0.5 | 1 |
| 2 | >8 | 97 | <5 | 5 |
| 3 | >8 | 76 | <5 | n.d. |
| 4 | 7 | 47 | 8 | 10 |
| 5 | >8 | 75 | <5 | n.d. |
| 6 | >8 | 55 | 20 | n.d. |
| 7 | >8 | 55 | >20 | 50 |
| 8 | >8 | 71 | >20 | 50 |
| 9 | >8 | 99 | 9 | 20 |

| | | | | |
|---|---|---|---|---|
| Assessed by mwPIFE. ^{∗∗}ATPlite assay in HCT116 cells treated for 5 days (Example 6) ^{∗∗∗}C-circle assay in HCT116 cells treated for 3 to 7 days (Example 4) | | | | |

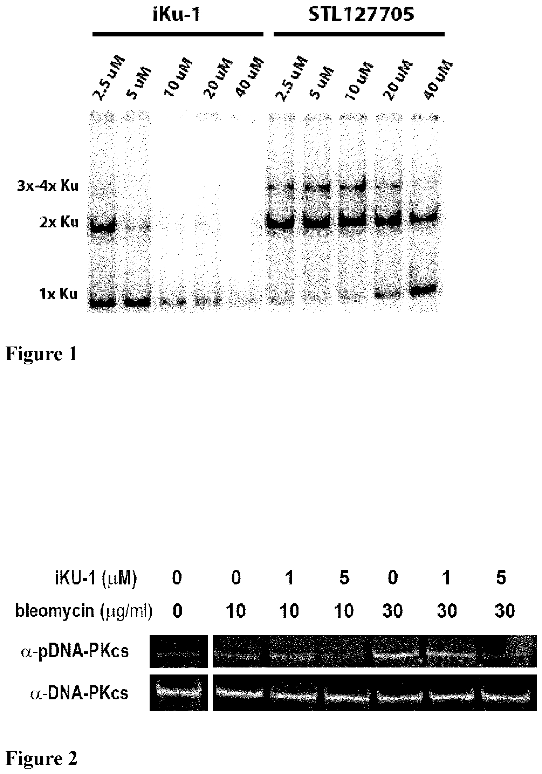

We also compared iKU-1 (Compound 1) and the published Ku inhibitor STL127705 (Weterings, E., Gallegos, A.C., Dominick, L.N., Cooke, L.S., Bartels, T.N., Vagner, J., Matsunaga, T.O., and Mahadevan, D. (2016). A novel small molecule inhibitor of the DNA repair protein Ku70/80. DNA Repair (Amst) 43, 98-106) in their efficiency to inhibit binding of Ki to double stranded DNA by electrophoretic mobility shift assay (Figure 1). A 98 bp long oligonucleotide that can bind up to four Ku complexes was used as a probe. The probe represents a 98bp cDNA fragment of *Arabidopsis thaliana* arginine/serine-rich splicing factor 35 amplified from Arabidopsis thaliana cDNA using Phusion DNA polymerase (blunt ends) and RS40-PTC-qF1 (5'-ATGAAAGCTGGGTTTGCTTTTG-3' (SEQ ID NO: 3)) and RS40-PTC-qR1 (5'-CGTCCCTTACGCCCAAAT-3' (SEQ ID NO: 4)) primers and end labeled with gamma[32P]ATP.

### Sequence of the 98bp DNA:

ATGAAAGCTGGGTTTGCTTTTGTATACATGGAAGATGAAAGGGATGCGGAAGATGCCATC
CGAGCACTTGACCGCTTTGAATTTGGGCGTAAGGGACG (SEQ ID NO: 5)

DNA (0.6 nM) in 10 µl EMSA buffer (35 mM Tris-Cl pH 7.9, 150 mM KCl, 1 mM EDTA, 0.1 mM Dithiothreitol, 5% Glycerol) was mixed with 10 µl of human Ku complex (100 nM in EMSA buffer) preincubated with the inhibitors for 5 min at room temperature, and incubated for additional 10 min. DNA associated with Ku complexes was seprated by native polyacrylamide gel electrophoresis (5% polyacrylamide 29:1 [Sigma-Aldrich], 0.5 TBE) at 160 V for 4 hrs. Gels were dried in gel dryer on 3MM paper, exposed to phospho-screen (GE Healthcare) and the autoradiograms were generated by scanning with Typhoon (GE Healthcare). The EMSA shows that iKU-1 is more potent inhibitor of Ku-DNA binding than STL127705 (Figure 1).

### Example 2

iKU-1 blocks activation of DNA-PKcs upon induction of DNA damage *in vivo* (Figure 2). HCT116 cells were pre-incubated with iKU-1 for 1 hr in McCoy's 5a medium supplemented with 10% Fetal Bovine Serum, 2 mM L-Glutamine, Penicilin/Streptomycin 200 U/l each. DNA breaks were induced by addition of bleomycin and incubated for additional 4 hrs. DNA damage triggers DNA-PKcs activity that leads to its autophosphorylation at the residue 2056. The phosphorylated DNA-PKcs was detected by Western blotting using anti-pDNA PKcs (phospho S2056) antibody [EPR5670] (Abcam, ab124918) and the total amount of DNA-PKcs with the anti-DNA PKcs antibody (BioRad, AHP318), both in 1:10000 dilution in TBS-T buffer overnight at 4°C. The primary antibody was detected by immunostaining with IRDye 800CW-conjugated Goat anti-Rabbit IgG secondary antibody (dilution 1:20000 in TBS-T). The signal was imaged using Odyssey Imager (Li-Cor). Whereas untreated sample shows high level of phosphorylated DNA-PKcs in the presence of bleomycin, iKU-1 blocked the phosphorylation even at 30 µg/ml concentration of bleomycin. The total level of DNA-PKcs was not altered by the treatment.

### Example 3

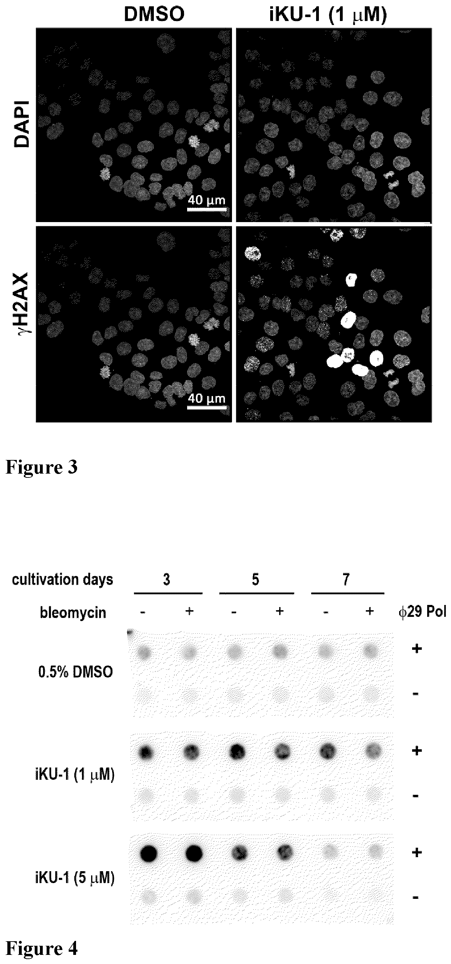

iKU-1 induces DNA damage in treated cells (Figure 3). HCT116 cells were incubated for 1 day in McCoy's 5a medium with 10% Fetal Bovine Serum, 2 mM L-Glutamine and Penicilin/Streptomycin 200 U/l each and supplemented with iKU-1. DNA damage induction was assessed by immunodetection of yH2AX in nuclei with specific antibody (Anti-phospho-Histone H2A.X (Ser139) Antibody, clone JBW301, Merck; dilution 1:500) and Alexa Fluor-conjugated secondary antibody (Goat anti-Mouse IgG (H+L) Highly Cross-Adsorbed Secondary Antibody, Alexa Fluor^{™} Plus 647, Invitrogen/Thermofisher; dilution 1:500). Cells were mounted in VECTASHIELD^{®} Antifade Mounting Medium with DAPI (Vector Laboratories; H-1200-10). γH2AX signal was detected by confocal laser scanning microscopy. iKU-1 treatment led to the formation of either nuclear foci or whole nuclear staining in a subset of cells.

### Example 4

Compounds 1-9 induce the formation of extrachromosomal circular telomeric DNA. HCT116 cell were incubated for 3, 5 or 7 days in McCoy's 5a medium with 10% Fetal Bovine Serum, 2 mM L-Glutamine and Penicilin/Streptomycin 200 U/l each and supplemented with Compound 1-9. C-circle assay that utilizes rolling circle amplification of circular DNA by φ29 polymerase was performed as described in Henson, J.D., Lau, L.M., Koch, S., Martin La Rotta, N., Dagg, R.A., and Reddel, R.R. (2017). The C-Circle Assay for alternative-lengthening-of-telomeres activity. Methods 114, 74-84. Amplified circular telomeric DNA was detected by dot blot hybridization with P³²-labeled telomeric DNA oligonucleotide (5'-CCCTAACCCTAACCCTAACCCTAA-3' (SEQ ID NO: 6)) (Figure 4). Concentration at which Ku inhibitors (Compounds 1-9) induced telomeric circles after 3 days of treatments is indicated in Table 1.

### Example 5

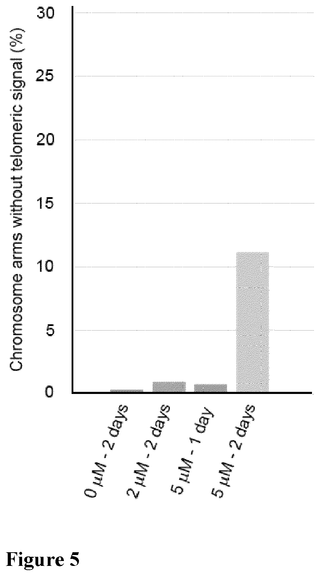

Presence of extrachromosomal circular telomeric DNA indicates rapid telomere shortening via excision of telomeric circles. To validate this prediction, we scored chromosome arms with no telomeric signal on metaphase chromosomes from iKU-1 treated cells by quantitative fluorescence *in situ* hybridization (Q-FISH). HCT116 cells were incubated for one or two days in McCoy's 5a medium with 10% Fetal Bovine Serum, 2 mM L-Glutamine and Penicilin/Streptomycin 200 U/l each and supplemented with iKU-1 (2 or 5 µM). Treated cells were incubated with colcemid (0.05 µg/ml) for 3 hours to increase a number of cells in metaphase. Cells were harvested and fixed in methanol and glacial acetic acid (3:1 v/v). Chromosome spreads on slides were hybridized with telomeric peptide nucleid acid probe TelC-Cy3 (Panagene, F1002) in 70% formamide, 0.5% blocking reagent (Roche) and 20 mM TRIS-Cl pH 7.4 according manufacturer's instruction. After washing, slides were counterstained with DAPI and mounted in VECTASHIELD^{®} Antifade Mounting Medium with DAPI (Vector Laboratories; H-1200-10). Fluorescence microscope with oil immersed 100× objective was used to detect telomeric signal at chromosome ends. This analysis showed that iKU-1 treated HCT116 cells exhibit increase incidence of telomere-free chromosome ends (Figure 5).

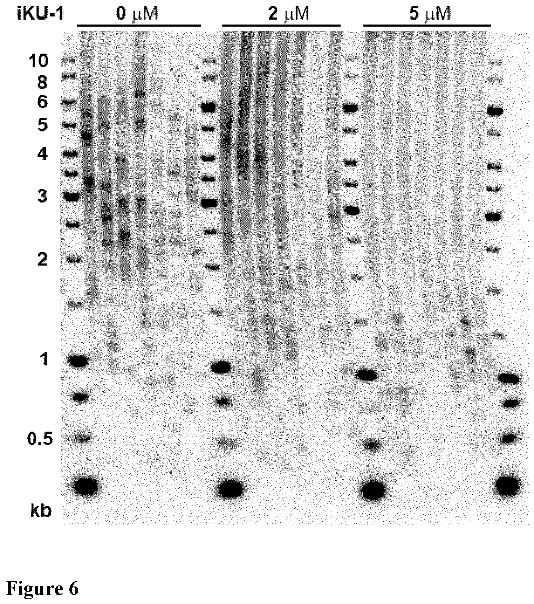

iKU-1 induces rapid shortening of telomeres (Figure 6). HCT116 cells were treated for two days with iKU-1 and telomere size was measured by Telomere Shortest Length Assay as described in Lai, T.P., Zhang, N., Noh, J., Mender, I., Tedone, E., Huang, E., Wright, W.E., Danuser, G., and Shay, J.W. (2017). A method for measuring the distribution of the shortest telomeres in cells and tissues. Nat Commun 8, 1356, whereby individual telomeres are PCR-amplified, separated by agarose gel electrophoresis and detected by Southern hybridization with P³²-labeled telomeric DNA oligonucleotide (5'-CCCTAACCCTAACCCTAACCCTAA-3' (SEQ ID NO: 6)). This assay showed relative decrease in the size of telomeres in iKU-1 treated samples in comparison with untreated control (Figure 6).

### Example 6

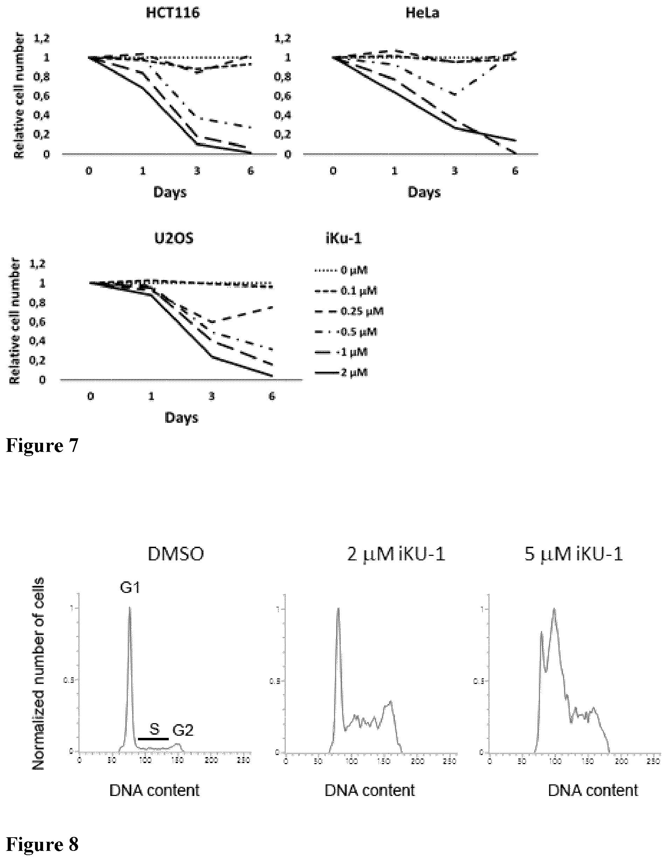

Compounds 1-9 inhibit proliferation of human tumor cell lines. HCT116, U2OS and HeLa cells were grown in McCoy's 5a medium supplemented with 10% Fetal Bovine Serum, 2 mM L-Glutamine and Penicilin/Streptomycin 200 U/l each in the presence of Compound 1-9. After growth at 37 °C, 5% CO2 for 3 days, monolayer of cells was detached using TrypLE Express (Gibco), cells were counted using 0.4% trypan blue stain and hemocytometer. 10,000 cells were seeded in 100 µl of media for 1 day and then treated with Compound 1-9 in concentrations from 0.1 to 2 µM. Number of viable cells was estimated using ATPlite kit (Perkin Elmer). IC₅₀ values for Compounds 1-9 are indicated in Table 1 for HTC116 cells. Growth curves of HTC116, U2OS and HeLa in the presence of different concentrations of iKU-1 (Compound 1) are shown in Figure 7.

### Example 7

Flow cytometry analysis that measures DNA content revealed that iKU-1 causes cell cycle arrest in S-phase (Figure 8). HCT116 cells were incubated for two days in McCoy's 5a medium supplemented with 10% Fetal Bovine Serum, 2 mM L-Glutamine and Penicilin/Streptomycin 200 U/l each in the presence of iKU-1, harvested and fixed in cold 70% ethanol. Cells were recovered in PBS solution and resuspended in 200 µl of Vindel solution containing propidium iodide and RNAse A, and nuclear DNA content was measured by flow cytometry (Beckton Dickinson FACS Verse). Accumulation of cells in S-phase is apparent already after two days of the iKU-1 treatment.

### Example 8

iKU-1 exhibits synergism with the telomerase inhibitor BIBR1532 (Boehringer Ingelheim) in HCT116 cancer cell line (Table 2). 10,000 HCT116 cells were seeded in 100 µl of McCoy's 5a medium supplemented with 10% Fetal Bovine Serum, 2 mM L-Glutamine and Penicilin/Streptomycin 200 U/l for 1 day in 96well plate and treated with 10 µM BIBR1532, 0.25 µM of iKU-1 or their combination for 4 days. Number of viable cells was measured by ATPlite kit (Perkin Elmer) and normalized to the treatment with 0.5% DMSO. The results presented in Table 2 show a synergistic effect of iKU-1 and BIBR1532 on the growth of HCT116 tumor cell line. Values in the table indicate relative growth assessed by ATPlite kit compared to the control sample treated with 0.5% DMSO after 4 days of treatment.

**Table 2**

| Cell line | BIBR1532 (10 µM) | iKU-1 (0.25 µM) | BIBR1532 + iKU-1 |
|---|---|---|---|
| HCT116 | 0.88 | 1.17 | 0.16 |
| U2OS | 0.88 | 0.81 | 0.51 |
| HeLa | 0.92 | 1.22 | 1.01 |

### Example 9

iKU-1 exhibits synergism with Bleomycin in cisplatin-sensitive human ovarian cancer cell line A2780 (Table 3). 2,000 A2780 cells were seeded in 100 µl of RPMI 1640 medium with 300 mg/l L-Glutamine and 10% Fetal Bovine Serum for 1 day in 96well plate and treated by 0.025 µg/ml of Bleomycin, 0.5 µM of iKu-1 or their combination for 7 days. Number of viable cells was measured by ATPlite kit (Perkin Elmer) and normalized to the treatment with 0.5% DMSO.

Results are shown in Table 3 which shows a synergistic effect of iKU-1 and Bleomycin on the growth of A2780 ovarian tumor cell line. Values in the table indicate relative growth assessed by ATPlite kit compared to the control sample treated with 0.5% DMSO after 7 days of treatment.

**Table 3**

| Cell line | Bleomycin (0.025 µg/ml) | iKU-1 (0.5 µM) | Bleomycin + iKu-1 |
|---|---|---|---|
| A2780 | 1.00 | 1.11 | 0.68 |

### Example 10

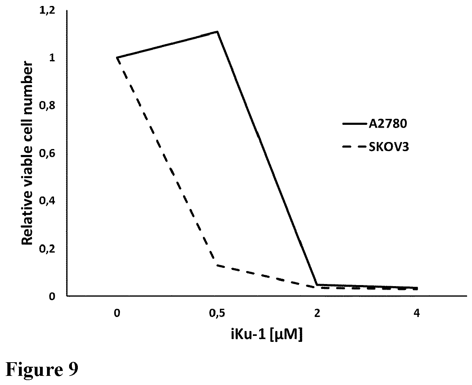

Cisplatin-resistant ovarian cancer cell line SKOV3 exhibits increased sensitivity to iKU-1. 2,000 SKOV3 and A2780 cells were seeded in 100 µl of McCoy's 5a or RPMI 1640 media, respectively, for one day and then incubated with iKU-1 for 7 days. Number of viable cells was measured by ATPlite kit (Perkin Elmer) and normalized to the treatment with 0.5% DMSO. SKOV3 cell are sensitive to iKU-1 at 0.5 µM concentration, whereas at this concentration A2780 cells do not show growth inhibition. The growth curves show that SKOV3 is 10-times more sensitive to iKU-1 than the A2780 cell line (Figure 9).

## Claims

1. Compound of general formula I 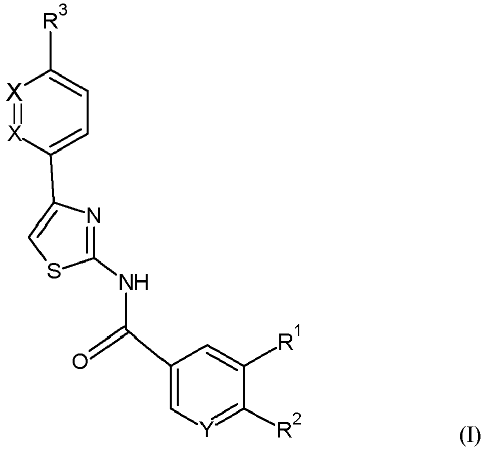 wherein
each of X is CH or N, wherein at most one X is N;
Y is CH or N;
R¹ is selected from H, halogen;
R² is selected from H, C1-C4 alkyl, C1-C4 alkylamino, C3-C6 cykloalkylamino;
R³ is selected from H, C1-C4 alkyl, -(CH₂)ₙ-NH-C(O)-(C1-C4 alkyl) wherein n=0 to 4;,
or pharmaceutically acceptable salts or solvates thereof,
for use in the treatment of tumor diseases.

2. Compound of general formula I for use according to claim 1, wherein at least one of X and Y is N; preferably one X is N and Y is N.

3. Compound of general formula I for use according to claim 1 or 2, wherein R¹ is selected from H, Br.

4. Compound of general formula I for use according to claim 1, wherein, the compound of general formula I is selected from 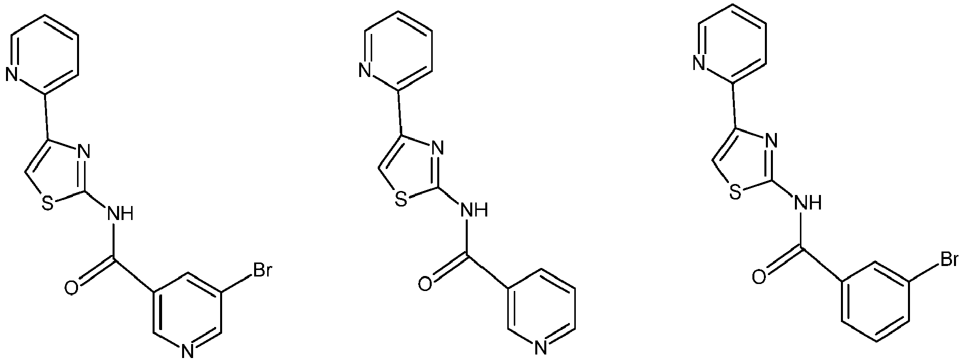 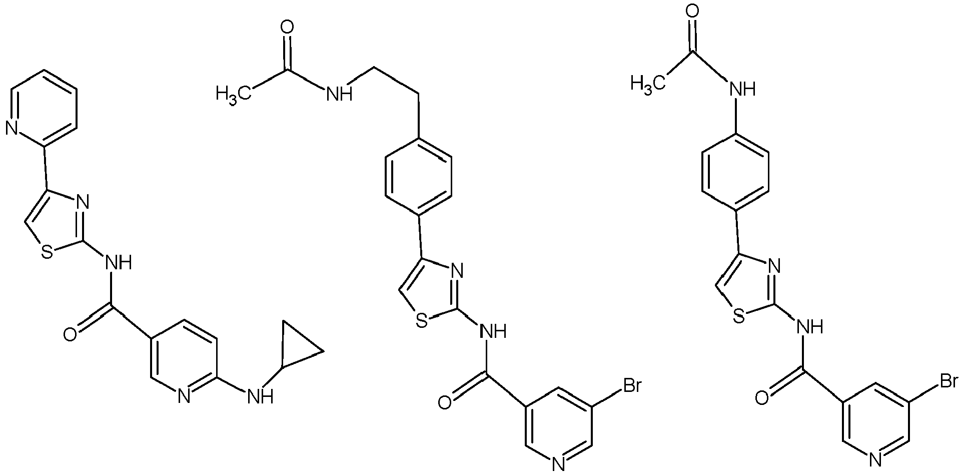 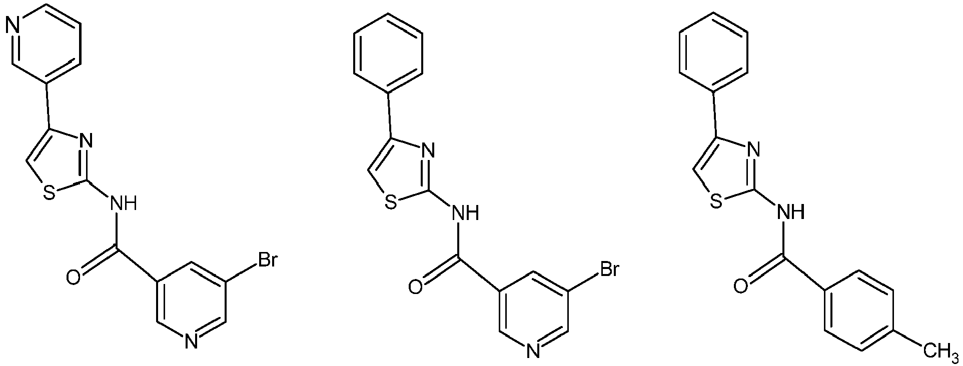 or pharmaceutically acceptable salts or solvates thereof.

5. Compound of general formula I for use according to any one of claims 1-4, wherein the tumor disease is a solid tumor with altered DNA damage response.

6. Compound of general formula I for use according to any one of claims 1-4, wherein the tumor disease is a tumor resistant to treatments with DNA damaging agent or to radiotherapy, or a tumor having mutations in genes of DNA damage response or repair.

7. Compound of general formula I for use according to any one of claims 1-4, wherein the tumor disease is a tumor resistant to at least one of bleomycin, cisplatin, topoisomerase II inhibitors, radiotherapy, or a tumors with mutations in at least one gene of BRCA1/2, ATM, TP53.

8. Compound of general formula I for use according to any one of claims 1-7, wherein the compound of general formula I is administered simultaneously or sequentially with radiotherapy or chemotherapy.

9. Compound of general formula I for use according to claim 8, wherein the chemotherapy is selected from DNA damage inducers, telomerase inhibitors, drugs inducing replication stress, inhibitors of other proteins involved in DNA repair and DNA damage response.

10. Compound of general formula I for use according to claim 8, wherein the chemotherapy is selected from BIBR1532, bleomycin, cis-platin, topoisomerase II inhibitors.
